# EUROPÄISCHE PATENTSCHRIFT

(11) **EP 1 108 444 B1**
(45) Veröffentlichungstag und Bekanntmachung des Hinweises auf die Patenterteilung: **23.04.2014**
(21) Anmeldenummer: 00126300.3
(22) Anmeldetag: 01.12.2000
(51) Int. Cl.: A61M 39/10, A61M 39/16, A61M 39/22

(54) **Sterilitäterhaltendes Konnektionssystem für medizinische Systeme und dessen Verwendung**
Sterility maintaining connection systems for medical systems and its utilisation
Système de connection stérile pour sytèmes médicaux ainsi que son utilisation

(30) Priorität: 14.12.1999 DE 19960226
(43) Veröffentlichungstag der Anmeldung: 20.06.2001
(73) Patentinhaber: Fresenius AG, 61352 Bad Homburg v.d.H. (DE)
(72) Erfinder: Lolachi, Houshang, Dr., Rockville Maryland 20852 (US); Neumann, Hans-Jürgen, Dr., 66606 St. Wendel (DE); Ebner, Siegfried, Dr., 66606 St.Wendel (DE)
(74) Vertreter: Laufhütte, Dieter

(56) Entgegenhaltungen:
- EP-A- 0 377 035
- EP-A- 0 555 927
- WO-A-99/44652
- FR-A- 2 506 162
- US-A- 3 749 271
- US-A- 5 270 003
- US-A- 5 792 120
- US-B1- 6 170 529

## Beschreibung

Die Erfindung betrifft ein steriles und/oder aseptisches Konnektionssystem und dessen Verwendung in medizinischen Systemen, speziell einem Set für die Zellseparation zum sterilen Verbinden weiterer Beutel an ein bestehendes Set. Die Erfindung betrifft im einzelnen zwei Konnektorteile, die steril und/oder aseptisch miteinander verbindbar sind, zum Überführen eines Fluids aus einem geschlossenen, sterilen Beutel in einen zweiten sterilen Beutel.

Im Stand der Technik sind zahlreiche Konnektoren bekannt, die zum Teil auch eine sterile Konnektion ermöglichen. Diese Konnektionssysteme sind notwendig geworden, um Kontaminationen bei der Überführung steril zu haltender Flüssigkeiten zu vermeiden. Dies geschieht beispielsweise bei der Überführung von Infusionslösungen, Dialyselösungen oder Blut bzw. Blutbestandteilen. Im Bereich der Infusionstechnik sind diverse Überleitsysteme bekannt, die meist aus einem Septum und der dazugehörigen Nadel bestehen. Bei der Überführung von Flüssigkeiten für die enterale Ernährung finden Spikes Verwendung, die ein größeres Flußvolumen besitzen.

Während die Sterilität der Spikes und Kanülen in der Infusion, d.h. der direkten Verwendung der Lösungen, meist ausreichend ist, ist an die Sterilität von zu lagerndem Blut oder Blutbestandteilen oder der Überführung von Dialyselösungen in das Peritoneum eine erhöhte Sterilanforderung gestellt, um eine Verkeimung zu verhindern. Derartige Lösungen bieten zum einen ein optimales Nährmedium für Bakterien und Pilze, zum anderen meist auch ein optimales Temperaturmilieu. Im Falle einer Peritoneallösung könnte ein Keim eine Peritonitis auslösen, also eine Bauchfellentzündung, die zu irreparablen Schäden führen kann. Im Falle von zu lagernden Produkten erhöht sich durch ideale Vermehrungsbedingung die Zahl der Keime derart, daß das Immunsystem bei einer entsprechenden Abwehr überfordert wird.

Daher ist es gefordert und üblich, bei der Übertragung von Blut von einem geschlossenen System in ein zweites System wie auch bei der Überführung von Lösungen in den Peritonealraum eine sterile Verbindung zu verwenden, die nachweislich an ihrer Konnektionsstelle von dem medizinischen Personal oder von den Patienten selbst auch nicht versehentlich berührt werden kann.

In den bekannten Sterilkonnektoren ist daher der Verschluß selbst, beispielsweise der Schraubverschluß, nach innen verlagert und von einem über den Verschluß gezogenen Überwurf vor Berührung geschützt.

Auch eine Kontamination durch Luftkontakt ist möglich, daher sind Konnektionssysteme bekannt, die eine innere Desinfektionsvorrichtung - beispielsweise getränkte Schwämmchen - aufweisen, so daß beim Zusammenstecken ein Desinfektionsmittel wie Jod-PVP freigesetzt und dadurch ein Verkeimen verhindert wird. Bei derartigen Systemen gelangt allerdings ein Teil des Desinfektionsmittels in die Schlauchverbindung. Im Hinblick darauf muß sichergestellt werden, daß das Desinfektionsmittel keinerlei Schäden verursachen kann.

Des Weiteren ist es bekannt, die Fluidverbindung erst dann zu schaffen, wenn das Konnektionssystem bereits steril verschlossen ist. In der WO 81101105 ist eine im Innern eines Schlauches befindliche Sollbruchstelle gezeigt, die durch Abbrechen des Plastikpins den Durchfluß ermöglicht. Dieses System wird allerdings mit einer fixen Schlauchverbindung hergestellt und danach sterilisiert, so daß keine Konnektion erforderlich ist.

In der DE 29607437 U beispielsweise ist eine medizintechnische Anschlußverbindung mit einem innen liegenden Gewinde beschrieben, deren Fluidverbindung durch Öffnen eines Brechsiegels ermöglicht wird, das in einem Innenkegel im axialen Abstand vom Einsteckende sitzt und daher nur von dem Außenkegel des Gegenkonnektors durchstoßen werden kann.

Ein besonders schnelles und sicheres Öffnen des Fluidweges wird durch Abbrechen realisiert, wie es beispielsweise in der EP 0 555 927 A2 beschrieben ist. In dieser Schrift ist ein Konnektionselement beschrieben, welches das Ende eines Schlauches dichtend verschließt. Eine Sollbruchlinie ermöglicht das paßgenaue Abbrechen des Verschlusses, um den Auslaß zu öffnen.

Auch in der WO 94/12224 ist ein Blutschlauchsystem gezeigt, welches über einen Zuführschlauch eine Klemme mit einer Sollbruchstelle zeigt. Mit Hilfe dieser Klemme kann der Schlauch alternativ von dem Einlaßbereich nur abgeklemmt werden oder durch Abbrechen an der Sollbruchstelle abgetrennt werden.

Aus der US 4,007,738 oder der EP 0555 927 ist es bereits bekannt bei einem Konnektorteil eine innenliegende Sollbruchstelle vorzusehen. Hier kann ein Endteil einer Verbindungsröhre an einer Sollbruchstelle, die innerhalb eines Verbindungsröhrchens, abgebrochen werden. Dieses abgebrochene Teil kann bei der dort vorgeschlagenen Lösung allerdings zu einem unerwünschten Zusetzen der Leitung führen.

Aus der US 5,743,892 ist ein Konnektionssystem bekannt, bei dem bereits ein Reservoir mit Desinfektionsmittel vorgesehen ist, das beim Zusammenstecken des weiblichen mit dem männlichem Konnektorteil bereits zur Desinfizierung der Verbindungsstelle dient.

Es ist Aufgabe vorliegender Erfindung, ein Konnektionssystem zu schaffen, welches hohen Sterilitätsanforderungen genügt und dennoch einfach und kostengünstig hergestellt werden kann. Die Aufgabe wird durch die im ersten Patentanspruch aufgeführten Merkmale gelöst.

Die erfindungsgemäße Konnektion besteht aus zwei Teilen, einem männlichen Konnektorteil und einem weiblichen Konnektorteil, die paßgenau ineinander gesteckt werden können.

Die Verbindung ist beispielsweise mittels eines Schraubgewindes herzustellen oder über Einrastvorrichtungen. Vorzugsweise allerdings werden die beiden Konnektorteile jedoch mit einem Kleber verbunden. In einer bevorzugten Ausführungsform sind die Konnektorteile irreversibel miteinander verbunden, um eine zusätzliche Sicherung zu erreichen.

Eine Markierung, wie z.B. ein Anschlag oder eine Einrastung, an den Konnektorteilen ermöglicht dabei festzustellen, ob die Verbindung formschlüssig erfolgt ist.

Dabei weisen beide Teile jeweils eine Sollbruchstelle auf, die nach formschlüssiger Verbindung möglichst übereinander positioniert sind. Auch wenn Toleranzen möglich sind, sollten die Sollbruchstellen bevorzugt direkt übereinander liegen. Diese Lage garantiert, daß beim Durchbrechen der Sollbruchstellen eine gemeinsame Bruchstelle entsteht, an der das zu überführende Fluid austreten kann.

Der Kontaktteil des Konnektionssystems ist dabei mit einem Klebstoff versehen, der eine desinfizierende Wirkung aufweist. Auf diese Weise kann ein Gewinde oder eine andere schwierig herzustellende Verbindung vermieden werden. Bei Verwendung eines Klebstoffes zur Verbindung existieren prinzipiell zwei Möglichkeiten.

Der Klebstoff kann sich zum einen auf dem männlichen Konnektorteil befinden. In diesem Falle wird die Oberfläche des männlichen Konnektorteiles beispielsweise in eine Klebstoffflüssigkeit getaucht oder der Klebstoff aufgetragen, so daß bei Verbindung ebenfalls eine vollständige Benetzung der Kontaktstelle auftritt. Wird der Konnektor bereits benetzt ausgeliefert, ist eine Schutzhülle vorgesehen.

Der Klebstoff kann sich zum anderen auch in dem weiblichen Konnektorstück befinden. Vorzugsweise ist der Klebstoff dabei in einem kleinen Vorratsgefäß, beispielsweise einer dünnen Polymerhülle oder mehreren Behältern wie z.B. Beads oder Microbeads innerhalb des Konnektorteiles eingeschlossen. Daher ist es möglich, einen in Kügelchen eingekapselten Ein- oder Mehrkomponentenklebstoff zu verwenden.

Beim Zusammenstecken des weiblichen und männlichen Konnektorteiles platzt die Klebstoffaufnahme und der Klebstoff wird durch den Preßsitz gleichmäßig über die gesamte Stelle verteilt. Es ist jedoch auch denkbar, daß der Klebstoff über die Innenseite verteilt ist und der Konnektor steril gegen die Außenseite mittels einer Schutzkappe, einer Schutzumhüllung oder einer aufgeklebten, durchstechbaren Schutzmembran oder eines Septums gegen die Außenseite abgeschlossen ist. Die Schutzmembran kann dabei sowohl an dem weiblichen als auch an dem männlichen Konnektorteil angebracht sein. In einer bevorzugten Ausführungsform weist der Klebstoff selbst eine desinfizierende Wirkung auf oder es wird dem Klebstoff zusammen oder in anderen Beads Desinfektionsmittel beigefügt. Beim Zusammenstecken oder eines durch Rastung oder Drehung erzwungenen Verschlusses platzen die o.g. Beads oder Kügelchen, die den Klebstoff und/oder das Desinfektionsmittel enthalten und vermischen sich, um die Oberflächen miteinander zu verbinden und/oder zu desinfizieren.

Weiterhin ist es vorteilhaft, wenn der Klebstoff schnell aushärtet, damit sich die Verbindung nicht wieder während des Einsatzes löst. Beispielsweise sei Cyanacrylat erwähnt, welches eine derartige Wirkung aufweist. Jedoch ist auch denkbar, daß der Klebstoff selbst nicht desinfizierend wirkt oder daß die Formschlüssigkeit über Gewinde oder Rastmittel erfolgt. Dann sollte zur Gewährleistung der Sterilität zusätzlich ein Desinfektionsmittel zwischen den Kontaktflächen des männlichen und weiblichen Konnektionsteiles eingefügt sein. Nachdem beide Konnektorteile ineinandergesteckt sind, verbreitet sich der Klebstoff auf der gesamten Kontaktstelle und härtet unmittelbar innerhalb weniger Sekunden aus.

Sodann wird die Verbindung geknickt, wodurch ein Bruch der verbundenen Konnektorteile an der Sollbruchstelle entsteht. Das abgebrochene Teilstück muß derart ausgestaltet sein, daß es im Schlauch steckenbleibt, aber dennoch keinen Verschluß gegen das durch den Schlauch fließende Fluid bildet. Dies wird dadurch erreicht, daß die Bruchstelle nicht zu weit an der Spitze des Konnektionssystems vorgesehen ist, da in diesem Fall das Bruckstück zu klein sein könnte und im Schlauch weiterbefördert wird. Es darf sich allerdings auch nicht zu weit an der breiten Seite des Konnektionssystems befinden, da sonst die Gefahr des Verstopfens gegeben ist. Aus diesen Gründen wählt man die Sollbruchstellen vorteilhafterweise etwa in der Mitte eines lang und dünn ausgestalteten Konnektionssystems.

Wie erwähnt befinden sich die Sollbruchstellen des männlichen und weiblichen Konnektorteiles im zusammengesteckten Zustand übereinander, so daß bei einem Bruch kein Klebstoff oder Desinfektionsmittel ins Innere des Konnektionssystems läuft. Selbstverständlich befindet sich die Bruchstelle im Inneren der zu verbindenden Schlauchenden, so daß der Schlauch selbst die Abdichtung gegen außen übernimmt.

Die erfindungsgemäße Konnektoranordnung wird beispielsweise in Beutel- und Schlauchanordnungen verwendet, in denen auf sterilem /aseptischem Wege eine Verbindung hergestellt werden muß. Diese Beutelsysteme können Blutbeutelsysteme sein, die oft auch einen Filter enthalten, wie einen Leukozytendepletionsfilter. Je nach Anwendung dieser Beutelsysteme ist die Beigabe eines Filters notwendig oder nicht, so daß man den Filter, einzelne Beutel oder Schlauchstücke beliebig frei zu einem fertigen Beutelsystem zusammenstecken kann.

Somit ist es natürlich auch möglich, daß zwei oder mehrfache Konnektion an einem Steckersystem gebildet werden, wie dies in der Figurenbeschreibung verdeutlicht wird.

Durch die erfindungsgmäße Konnektionsanordnung kann somit das Kontaminationsrisiko auf einfachem Wege und sicher vermieden werden. Dies betrifft zum einen das im Innern fließende Medium wie beispielsweise Blut, da weder Klebstoff oder Desinfektionsmittel ins Blut gelangen kann, noch Keime, die die Lagerung und Verwendung der Blutkonserve unmöglich machen würden. Durch die sichere und festverklebte Verbindung kann auch zum anderen kein Blut in die Umgebung gelangen, so daß auch hierdurch eine Verunreinigung durch beispielsweise Viren nicht weiter getragen werden kann.

Vorzugsweise bestehen die Konnektionsteile aus Kunststoff, z.B. Polycarbonat, Acrylnitril-Butadien-Styrole, Polyethylene, Polypropylene, Polystyrole, Polymethylmethacrylate, Polysulfone oder Methylmethacrylat-Butadien-Styrole oder Methylmethacrylat-Acrylnitril-Butadien-Styrole, die mit dem sie berührenden Schlauchmaterial kompatibel sein sollen, mit dem Klebstoff, wie auch mit den durch sie fließenden Medien. Im Falle von Blut oder Blutbestandteilen bietet sich ein PVC-Schlauch oder auch ein Polyolefinschlauch an.

Vorteilhaft sind für das männliche bzw. weibliche Konnektorteil Schutzkappen vorgesehen, die mit den jeweiligen Elementen verbindbar sind und diese steril halten und vor Beschädigungen schützen, bis sie zusammengesteckt werden.

Im folgenden wird anhand der Figurenbeschreibung eine bevorzugte Ausführungsform des Konnektionssystems beschrieben.
- Figur 1: zeigt das Konnektionssystem im dekonnektierten Zustand.
- Figur 2: zeigt das Konnektionssystem im konnektierten Zustand.
- Figur 3: zeigt eine Ausführungsform, in der der Klebstoff und/oder das Desinfektionsmittel in vielen Mikrokügelchen vorliegt.
- Figur 4: zeigt das Konnektionssystem mit zwei Beads.
- Figur 5: zeigt eine Membran innerhalb des Konnektionssystems als Abschluß.
- Figur 6: zeigt verschiedene Formen des Konnektionssystems im Durchschnitt,
- Figur 7 a, b und c: zeigen Mehrfachkonnektionssysteme, a/b ohne und c mit Filter,
- Figur 8, 8a: zeigen eine Seitenansicht und einen Schnitt durch das männliche Teil des Konnektionssystems, das mit einer Schutzkappe verbunden ist und
- Figur 9, 9a: zeigt eine Seitenansicht und einen Schnitt durch das weibliche Teil des Konnektionssystems, mit welchem eine Schutzkappe verbunden ist.

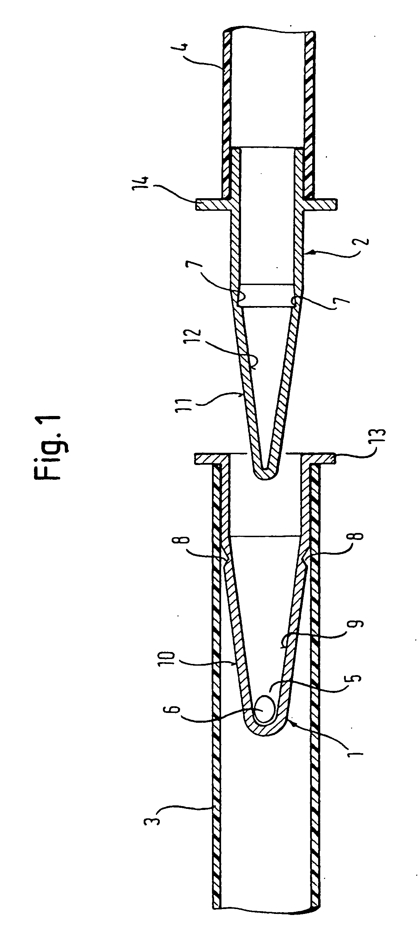

In Figur 1 ist ein Konnektionssystem gezeigt, das zur Verbindung zweier Schlauchabschnitte 3, 4 dient, wobei das weibliche und das männliche Konnektorteil 1, 2 getrennt vorliegen. Der erste Schlauchabschnitt 3 nimmt einen Teil des weiblichen Konnektorteiles 1 auf, so daß die Spitze im Schlauchinneren zu liegen kommt. Im Innenbereich 5 der Spitze wiederum befindet sich ein Vorrat an aseptischem Klebstoff 6.

Das weibliche Konnektorteil weist eine Sollbruchstelle 8 auf, die sich durch eine Materialeinsparung ergibt, die sich kreisrund um den Konnektor erstreckt. Im vorliegenden Fall befindet sich die Materialeinsparung in Form einer Kreiskerbe im Außenbereich 10, so daß die innenliegende Fläche 9 eine glatte Oberfläche besitzt.

Das männliche Konnektorteil 2 ist an seinem Konnektionsende an einem Schlauchende 4 irreversibel befestigt, so daß die Spitze des Konnektors frei sichtbar ist. Auch das männliche Konnektorteil weist eine Sollbruchstelle 7 auf, die sich im Gegensatz zu seinem Gegenstück im Innenbereich 12 befindet, so daß die äußere Oberfläche 11 ebenfalls glatt ist. Die Lage der Sollbruchstelle ist in der vorliegenden Figur lediglich skizzenhaft dargestellt. Wie bereits im Text beschrieben, muß gewährleistet sein, daß das abgebrochene Endstück sich im Schlauch verkeilt, ohne den Durchfluß zu verhindern.

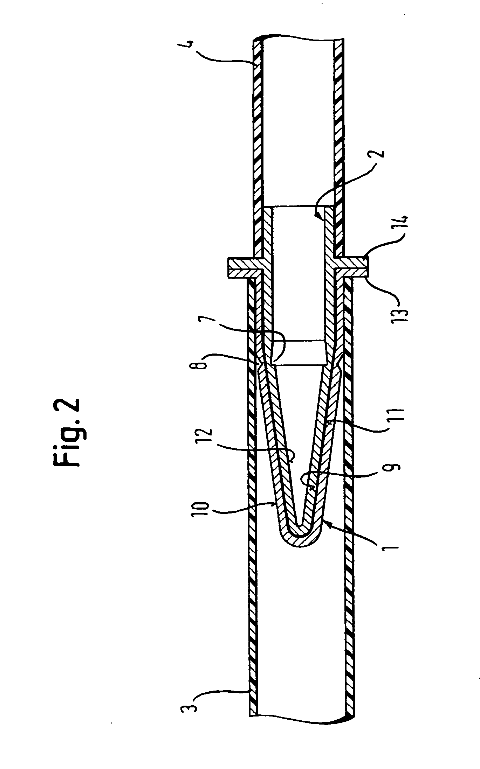

Im konnektierten Zustand - wie in Figur 2 gezeigt - kommen die Sollbruchstellen übereinander zu liegen und ergeben dadurch eine einzige gemeinsame Sollbruchstelle. Der Klebstoff hat sich durch den Preßsitz gleichmäßig über die Flächen 9 und 11 verteilt und verbindet die Konnektorteile formschlüssig. Vorteilhafterweise besitzen beide Konnektionsteile 1, 2 einen Anschlag 13, 14, der dem Benutzer anzeigt, ob die beiden Konnektorsteile formschlüssig aufeinander gepreßt sind. Im geschlossenen Zustand darf kein Spalt zwischen dem Anschlag 13 des weiblichen Konnektorteils und dem Anschlag 14 des männlichen Konnektorteils mehr zu erkennen sein. Auch kann eine Verkantung genau gesehen werden.

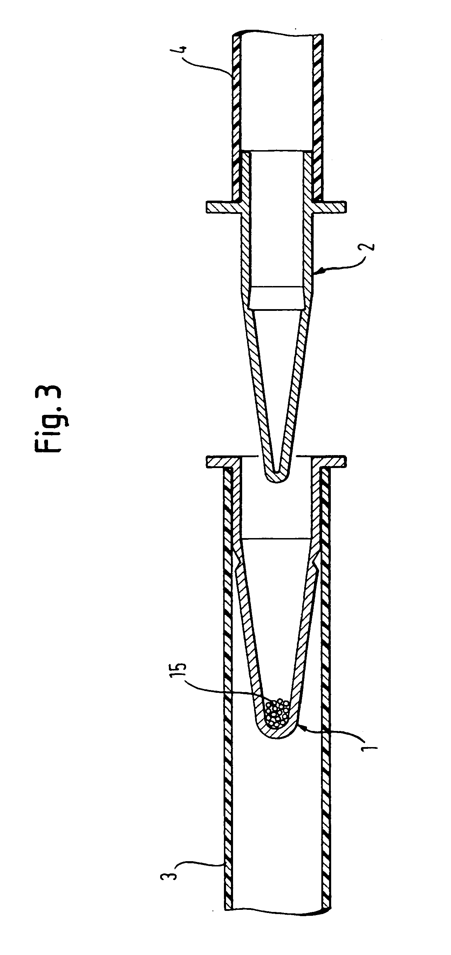

Figur 3 zeigt das Konnektorsystem mit vielen kleinen Beads 15, die jeweils entweder Klebstoff oder Desinfektionsmittel enthalten.

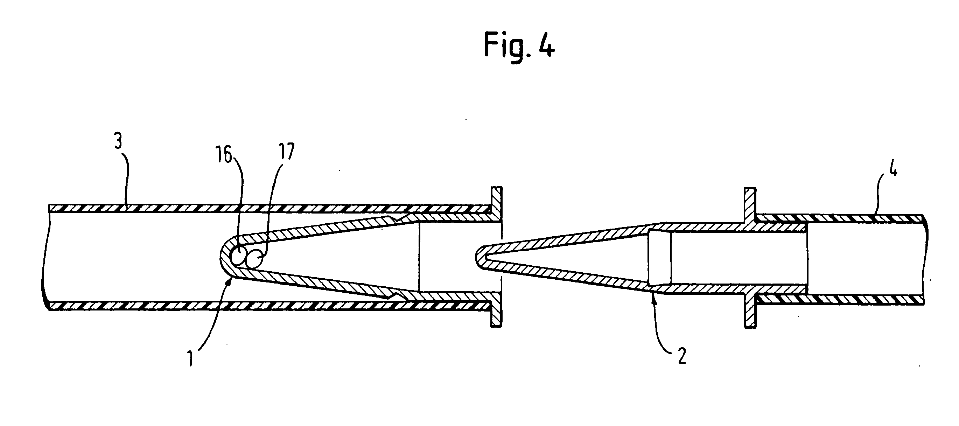

In Figur 4 sind lediglich zwei Behältnisse 16 und 17 gezeigt, die jeweils eine Komponente eines Zweikomponentenklebers enthalten sollen. Selbstverständlich sind die Ausführungsformen jedoch nicht auf zwei Komponenten beschränkt.

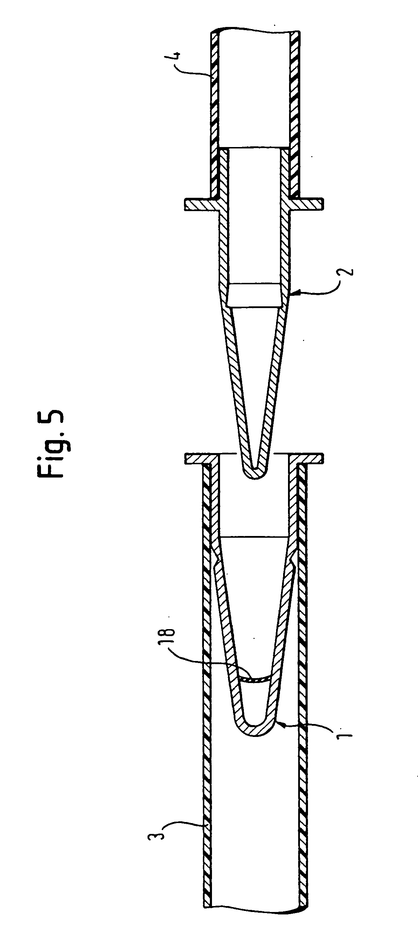

Figur 5 offenbart eine Membran 18 oder ein Septum innerhalb des weiblichen Konnektionssystems, welches mit Hilfe des männlichen Teils durchstochen werden kann und dadurch das abgeschlossene Mittel freigesetzt werden kann.

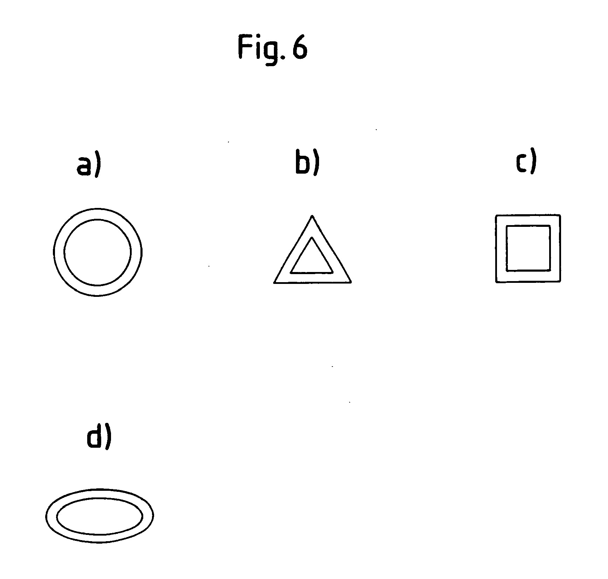

In Figur 6 sind die Geometrien des Konnektionssystems gezeigt, wobei a) kreisförmig, b) dreieckig, c) viereckig und d) elliptisch ist.

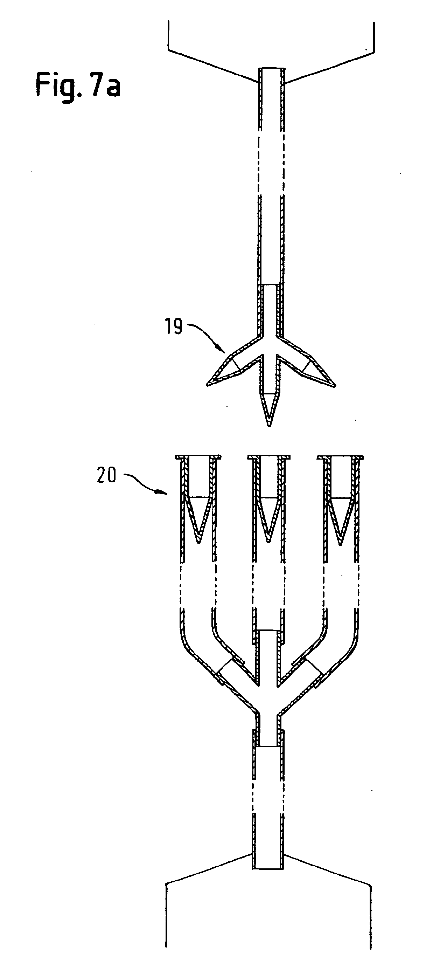

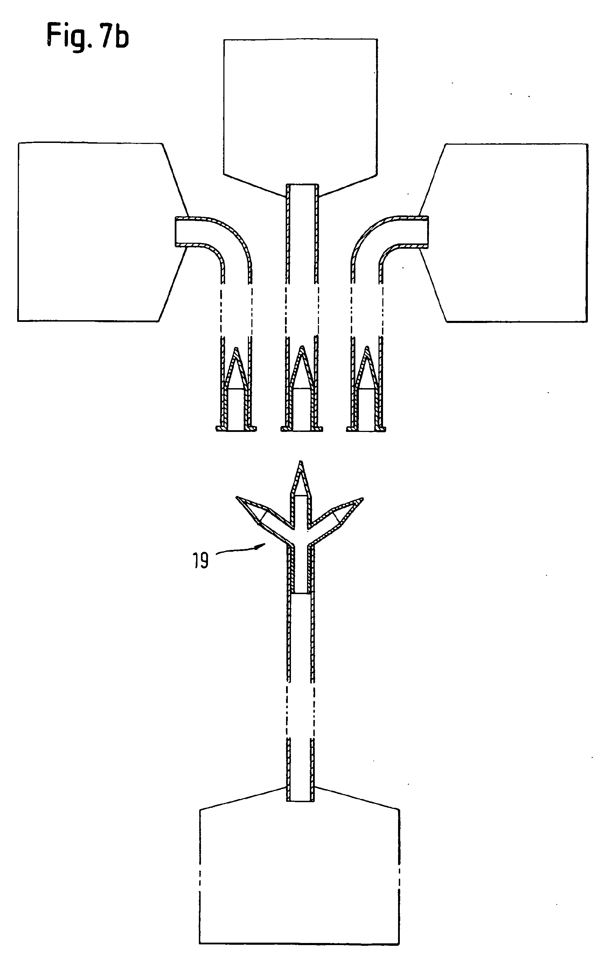

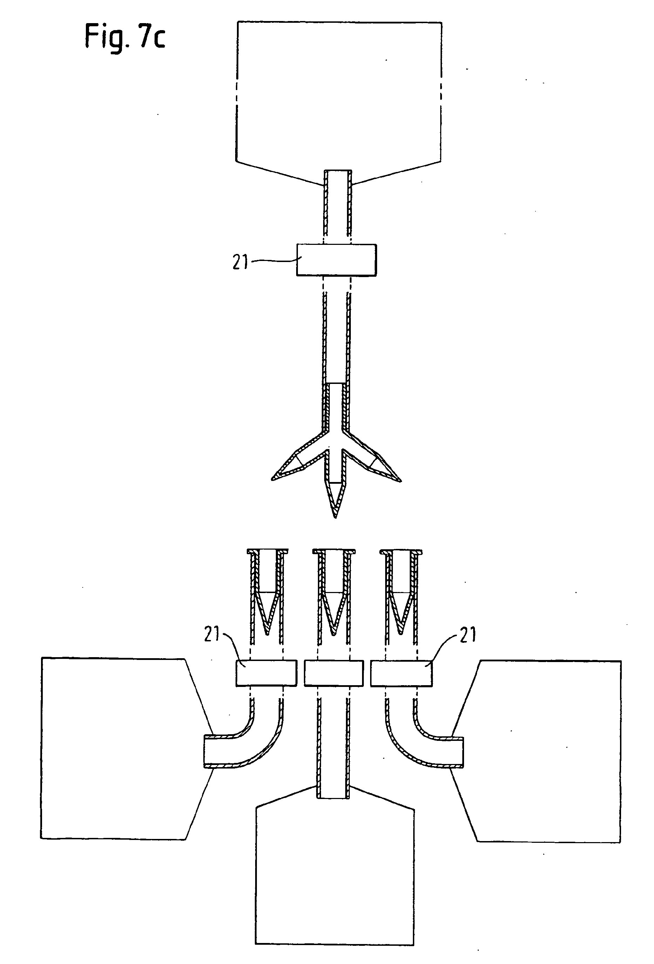

Die Figuren 7 zeigen jeweils schematisch dargestellt die Mehrfachkonnektionen. Figur 7a zeigt einen Mehrfachstecker 19 am männlichen Teil sowie an einem weiblichen Teil 20, während in 7b der Mehrfachstecker 19 einzelnen anderen weiblichen Gegenstücken zugeordnet ist. Die Figur 7c ist mit Filtern 21, vorzugsweise zur Elimination von Leukozyten, ergänzt.

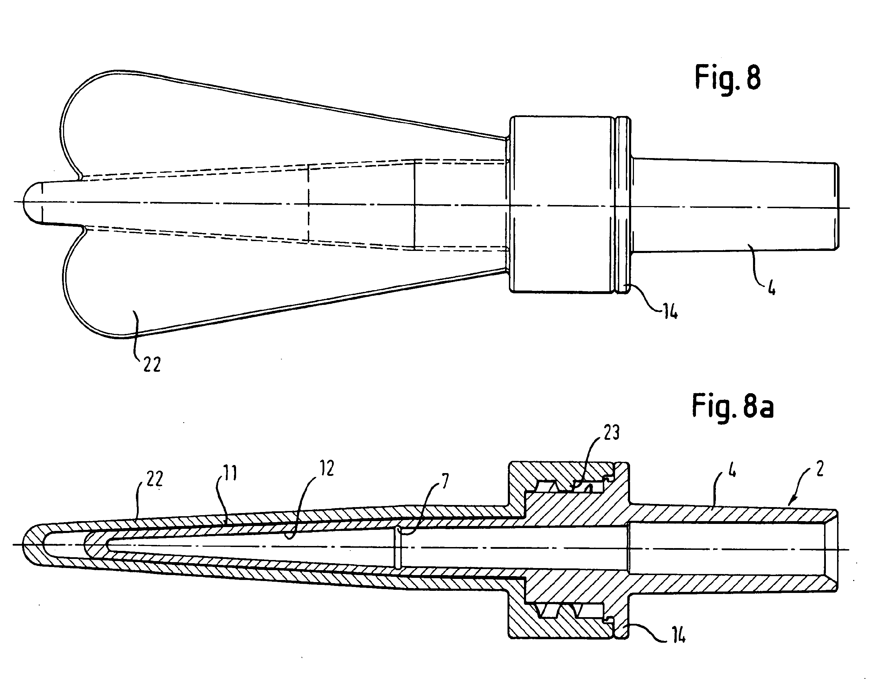

Die Figuren 8 und 8a zeigen das männliche Konnektorteil 2 entsprechend dem zuvor beschriebenen Aufbau, mit dem eine Schutzkappe 22 über die entsprechende Schraubverbindung 23 verbunden ist.

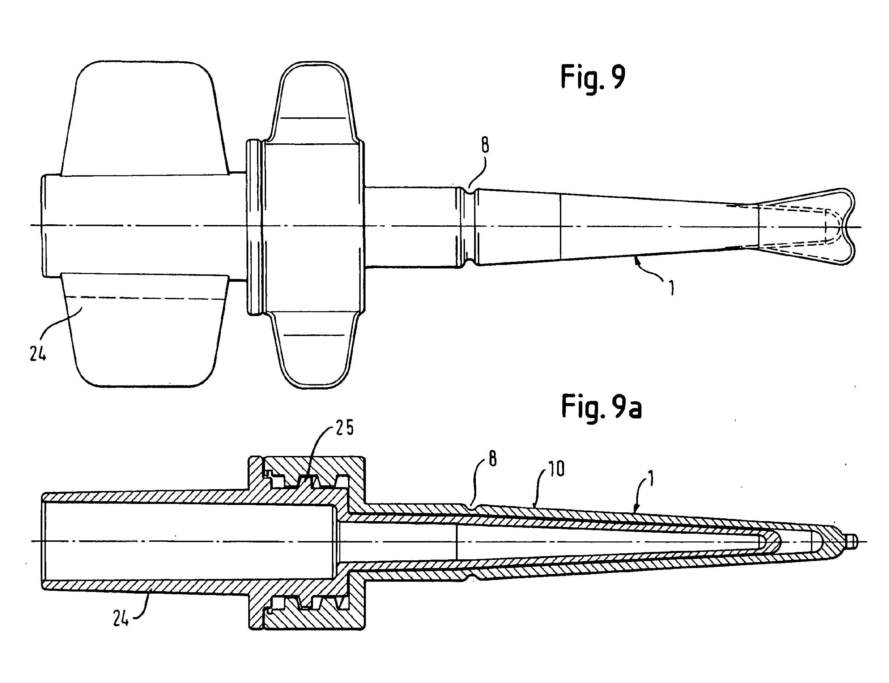

Ähnlich ist in den Figuren 9 und 9a das weibliche Konnektorteil 1, das ebenfalls dem zuvor beschriebenen Aufbau entspricht, mit einer Schutzkappe 24 verbunden. Die Schutzkappe 24 entspricht in ihrer Form dem männlichen Konnektorteil 1. Die Schutzkappen 23 und 24 weisen, wie in den Figuren 8 und 9 dargestellt, flügelartige Fortsätze auf, die eine Handhabung der Schutzkappen beim Lösen und Verschließen erleichtern.

Die Schutzkappen 22 und 24 dienen zum Schutz des männlichen Konnektorteils 2 bzw. weiblichen Konnektorteils 1 und weisen Gewinde 23 bzw. 25 auf, um mit den männlichen bzw. weiblichen Konnektorteilen sicher verbunden zu sein, so daß ein Kontakt der Konnektorteile mit der Umgebung bis zum Lösen von den Schutzkappen 22 und 24 sicher verhindert wird. Die Schutzkappen 22 und 24 bieten darüber hinaus einen Schutz vor extern auftretenden Kräften bzw. intern im Leitungssystem auftretenden Drücken. Üblicherweise werden die Schutzkappen 22 und 24 erst unmittelbar vor dem Zusammenfügen des weiblichen und des männlichen Konnektorteils von der Bedienperson entfernt.

## Patentansprüche

1. Konnektionssystem zum Verbinden insbesondere zweier oder mehrerer steriler Systeme, umfassend mindestens ein männliches Konnektorteil (2), das ein geschlossenes Ende eines sterilen, fluidführenden Systems bildet, und mindestens ein weibliches Konnektorteil (1), welches das geschlossene Ende eines zweiten sterilen fluidführenden Systems bildet, die aseptisch miteinander verbindbar sind,
**dadurch gekennzeichnet,**
**dass** die beiden Konnektorteile (1, 2) an ihren sich berührenden Kontaktflächen (9, 11) ein Desinfektionsmittel aufweisen, so dass die Verbindung antiseptisch ist, wobei die beiden Konnektorteile (1, 2) jeweils eine Sollbruchstelle (7, 8) aufweisen, die im zusammengefügten Zustand der beiden Konnektorteile (1, 2) übereinanderliegen, so dass sie eine gemeinsame Sollbruchstelle bilden und zusammen abbrechbar sind, so dass kein Eindringen von Desinfektionsmittel in das Innere des Konnektionssystems möglich ist, und wobei sich die Söllbruchstelle im Inneren des fluidführenden Systems befindet.

2. Konnektionssystem nach Anspruch 1, **dadurch gekennzeichnet, dass** die beiden Konnektorteile (1, 2) formschlüssig miteinander verbindbar sind.

3. Konnektionssystem nach einem der Ansprüche 1 oder 2, **dadurch gekennzeichnet, dass** die Konnektorteile (1, 2) mittels eines Schraubgewindes verbindbar sind.

4. Konnektionssystem nach einem Ansprüche 1 oder 2, **dadurch gekennzeichnet, dass** die Konnektorteile (1, 2) mittels einer Einrastverbindung verbindbar sind.

5. Konnektionssystem nach einem Ansprüche 1 oder 2, **dadurch gekennzeichnet, dass** die Konnektorteile (1, 2) mittels einer Haftverbindung verbindbar sind.

6. Konnektionssystem nach Anspruch 5, **dadurch gekennzeichnet, dass** die Haftverbindung ein schnell härtender Klebstoff ist.

7. Konnektionssystem nach einem der vorhergehenden Ansprüche, **dadurch gekennzeichnet, dass** das Desinfektionsmittel haftverbindene Eigenschaften besitzt.

8. Konnektionssystem nach einem der vorhergehenden Ansprüche, insbesondere nach Anspruch 7, **dadurch gekennzeichnet, dass** das Desinfektionsmittel ein schnell härtender Klebstoff ist.

9. Konnektionssystem nach einem oder mehreren der vorhergehenden Ansprüche, **dadurch gekennzeichnet, dass** Cyanacrylat zur Verbindung der beiden Konnektorteile (1, 2) vorgesehen ist.

10. Konnektionssystem nach einem oder mehreren der vorhergehenden Ansprüche, **gekennzeichnet durch** eine Schutzkappe (22), das in seiner Außenkontur an das männliche Konnektorteil angepaßt ist.

11. Konnektionssystem nach einem oder mehreren der vorhergehenden Ansprüche **gekennzeichnet durch** eine Schutzkappe (24) in Form eines männlichen Konnektorteils, das zum Schutz des weiblichen Konnektorteils (1) mit diesem verbindbar ist.

12. Weibliches Konnektorteil (1) mit einer Sollbruchstelle (8), welches das geschlossene Ende eines fluidführenden Systems bildet, geeignet zum Verbinden mit einem männlichen Konnektorteil (2) mit einer Sollbruchstelle (7), welches das Ende eines zweiten fluidführenden Systems bildet,
sodaß im verbundenen Zustand der beiden Konnektorteile (1, 2) die jeweiligen Sollbruchstellen (7, 8) übereinander liegen, und wobei die beiden Konnektorteile (1, 2) an ihren sich berührenden Kontaktflächen (9, 11) ein Desinfektionsmittel aufweisen.

13. Verwendung eines Konnektionssystems gemäß Anspruch 1 zur sterilen Überführung von Fluid innerhalb eines Beutelsystems, mit mindestens zwei Beuteln und einem Schlauchsystem.

14. Verwendung eines Konnektionssystems nach Anspruch 13 zur sterilen Überführung von biologischen oder medizinischen Fluiden innerhalb eines Beutelsystems, mit mindestens zwei Beuteln und einem Schlauchsystem.

15. Verwendung eines Konnektionssystems nach einem der Ansprüche 13 oder 14 in einem sterilen Blutbeutelsystem zur sterilen Überführung von Blut oder Blutbestandteilen.

16. Verwendung eines Konnektionssystems nach einem der Ansprüche 14 bis 15 in einem Beutel- und Schlauchsystem mit mindestens einem Filterelement zur sterilen Überführung von Blut oder Blutbestandteilen.

17. Verwendung eines Konnektionssystems nach Anspruch 13 in einem Beutelsystem zur sterilen Überführung von Infusions- oder Dialyselösung.

## Claims

1. A connection system for connecting sterile systems, in particular two or more sterile systems, comprising at least one male connector part (2) which forms a closed end of a sterile, fluid-conducting system and at least one female connector part (1) which forms the closed end of a second sterile fluid-conducting system, said systems being aseptically connectable to one another,
**characterised in that**
the two connector parts (1, 2) have a disinfectant at their contacting contact surfaces (9, 11) so that the connection is antiseptic, with the two connector parts (1, 2) each having a predetermined breaking point (7, 8), said breaking points being disposed above one another in the connected state of the two connector parts (1, 2) so that they form a common predetermined breaking point and can be broken off together so that no penetration of disinfectant into the interior of the connection system is possible, and with the desired breaking point being located at the interior of the fluid-conducting system.

2. A connection system in accordance with claim 1, **characterised in that** the two connector parts (1, 2) are connected to one another in shape-matched form.

3. A connection system in accordance with one of the claims 1 or 2, **characterised in that** the connector parts (1, 2) are connectable by means of a screw thread.

4. A connection system in accordance with one of the claims 1 or 2, **characterised in that** the connector parts (1, 2) are connectable by means of a latch-in connection.

5. A connection system in accordance with one of the claims 1 or 2, **characterised in that** the connector parts (1, 2) are connectable by means of an adhesive connection.

6. A connection system in accordance with claim 5, **characterised in that** the adhesive connection is a fast-setting adhesive.

7. A connection system in accordance with one of the preceding claims, **characterised in that** the disinfectant has bonding properties.

8. A connection system in accordance with one of the preceding claims, in particular in accordance with claim 7, **characterised in that** the disinfectant is a fast-setting adhesive.

9. A connection system in accordance with one or more of the preceding claims, **characterised in that** cyanoacrylate is provided for connecting the two connector parts (1, 2).

10. A connection system in accordance with one or more of the preceding claims, **characterised by** a protective cap (22) whose outer contour is adapted to the male connector part.

11. A connection system in accordance with one or more of the preceding claims, **characterised by** a protective cap (24) in the form of a male connector part which can be connected to the female connector part (1) for the protection thereof.

12. A female connector part (1) having a predetermined breaking point (8) which forms the closed end of a fluid-conducting system, suitable for connection to a male connector part (2) having a predetermined breaking point (7) which forms the end of a second fluid-conducting system so that the respective predetermined breaking points (7, 8) are disposed above one another in the connected state of the two connector parts (1, 2), and wherein the two connector parts (1, 2) have a disinfectant at their contacting contact surfaces (9, 11).

13. Use of a connection system in accordance with claim 1 for the sterile conveying of fluid within a bag system having at least two bags and one hose system.

14. Use of a connection system in accordance with claim 13 for the sterile conveying of biological or medical fluids within a bag system having at least two bags and one hose system.

15. Use of a connection system in accordance with one of the claims 13 or 14 in a sterile blood bag system for the sterile conveying of blood or blood components.

16. Use of a connection system in accordance with one of the claims 14 to 15 in a bag and hose system having at least one filter element for the sterile conveying of blood or blood components.

17. Use of a connection system in accordance with claim 13 in a bag system for the sterile conveying of infusion solution or dialysis solution.

## Revendications

1. Système de connexion destiné à raccorder en particulier deux ou plusieurs systèmes stériles, comprenant au moins une pièce de connecteur mâle (2), qui forme une extrémité fermée d'un système stérile transportant un fluide, et au moins une pièce de connecteur femelle (1), qui forme l'extrémité fermée d'un second système stérile transportant un fluide, qui peuvent être raccordées l'une à l'autre de manière aseptique,
**caractérisé en ce que**
les deux pièces de connecteur (1, 2) présentent un agent désinfectant sur leurs surfaces de contact (9, 11) en prise, de sorte que le raccordement est antiseptique, les deux pièces de connecteur (1, 2) présentant respectivement un point destiné à la rupture (7, 8), ces deux points destinés à la rupture se superposant à l'état assemblé des deux pièces de connecteur (1, 2), de sorte qu'ils forment un point destiné à la rupture commun et qu'ils peuvent être rompus ensemble, de sorte qu'aucune pénétration d'agent désinfectant à l'intérieur du système de connexion ne soit possible, et le point destiné à la rupture se trouvant à l'intérieur du système transportant un fluide.

2. Système de connexion selon la revendication 1, **caractérisé en ce que** les deux pièces de connecteur (1, 2) peuvent être raccordées l'une à l'autre par coopération de formes.

3. Système de connexion selon l'une des revendications 1 ou 2, **caractérisé en ce que** les pièces de connecteur (1, 2) peuvent être raccordées au moyen d'un filet de vissage.

4. Système de connexion selon l'une des revendications 1 ou 2, **caractérisé en ce que** les pièces de connecteur (1, 2) peuvent être raccordées au moyen d'un raccord par encliquetage.

5. Système de connexion selon l'une des revendications 1 ou 2, **caractérisé en ce que** les pièces de connecteur (1, 2) peuvent être raccordées au moyen d'une liaison par adhérence.

6. Système de connexion selon la revendication 5, **caractérisé en ce que** la liaison par adhérence est une matière adhésive à durcissement rapide.

7. Système de connexion selon l'une des revendications précédentes, **caractérisé en ce que** l'agent désinfectant possède des caractéristiques d'adhérence.

8. Système de connexion selon l'une des revendications précédentes, en particulier selon la revendication 7, **caractérisé en ce que** l'agent désinfectant est une matière adhésive à durcissement rapide.

9. Système de connexion selon l'une ou plusieurs des revendications précédentes, **caractérisé en ce que** du cyanoacrylate est destiné à raccorder les deux pièces de connecteur (1, 2).

10. Système de connexion selon l'une ou plusieurs des revendications précédentes, **caractérisé par** un capuchon protecteur (22) dont le contour extérieur est adapté à la pièce de connecteur mâle.

11. Système de connexion selon l'une ou plusieurs des revendications précédentes, **caractérisé par** un capuchon protecteur (24) sous forme d'une pièce de connecteur mâle, qui peut être raccordé à la pièce de connecteur femelle (1) pour la protection de celle-ci.

12. Pièce de connecteur femelle (1) comprenant un point destiné à la rupture (8), qui forme l'extrémité fermée d'un système transportant un fluide, adapté pour le raccordement avec une pièce de connecteur mâle (2) comprenant un point destiné à la rupture (7), qui forme l'extrémité d'un second système transportant un fluide, de sorte qu'à l'état raccordé des deux pièces de connecteur (1, 2), les points respectifs destinés à la rupture (7, 8) sont superposés, et les deux pièces de connecteur (1, 2) présentant un agent désinfectant sur leurs surfaces de contact (9, 11) en prise.

13. Utilisation d'un système de connexion selon la revendication 1 pour l'acheminement stérile de fluide à l'intérieur d'un système de poches, comprenant au moins deux poches et un système de tuyaux.

14. Utilisation d'un système de connexion selon la revendication 13 pour l'acheminement stérile de fluides biologiques ou médicaux à l'intérieur d'un système de poches, comprenant au moins deux poches et un système de tuyaux.

15. Utilisation d'un système de connexion selon l'une des revendications 13 ou 14 dans un système de poches de sang stérile pour l'acheminement stérile de sang ou de constituants sanguins.

16. Utilisation d'un système de connexion selon l'une des revendications 14 à 15 dans un système de poches et de tuyaux, comprenant au moins un élément filtrant pour l'acheminement stérile de sang ou de constituants sanguins.

17. Utilisation d'un système de connexion selon la revendication 13 dans un système de poches pour l'acheminement stérile d'une solution de perfusion ou de dialyse.
